Europäisches Patentamt

European Patent Office

Office européen des brevets

(19)

(11) Publication number: 0 357 400
A2

# EUROPEAN PATENT APPLICATION

(12)

(21) Application number: 89308774.2

(22) Date of filing: 30.08.89

(51) Int. Cl.⁵: G 01 N 33/52
C 12 Q 1/60, C 12 Q 1/28

(30) Priority: 30.08.88 US 238775   08.03.89 US 320414

(43) Date of publication of application:
07.03.90 Bulletin 90/10

(84) Designated Contracting States:
AT BE CH DE ES FR GB GR IT LI LU NL SE

(71) Applicant: CHOLESTECH CORPORATION
3100 Diablo Avenue
Hayward California 94545 (US)

(72) Inventor: Hewett, Gary E.
14735 Saltamontes
Los Altos Hills California 94022 (US)

Mielke, Steven T.
319 I Street
Fremont California 94536 (US)

Blunt, Judith A.
33487 Caliban Drive
Fremont California 94536 (US)

(74) Representative: Goldin, Douglas Michael et al
J.A. KEMP & CO. 14, South Square Gray's Inn
London WC1R 5EU (GB)

(54) Self-corrected assay device and method.

(57) An assay device (10) and method for determining the concentration of an analyte in a fluid sample. The device includes at least two reaction zones (18, 20) which receive a fluid sample, and each reaction zone includes the same signal-generating components for converting an intermediate reaction product to a detectable reaction product. Where the intermediate product is $H_2O_2$, the signal-generating components may further include a trapping compound which competes with the substrate of the detectable signal product, to produce a silent reaction product, proportionately reducing the amount of detectable reaction product produced. In an embodiment of the device containing two reaction zones, the signal generated in the second zone is used to construct a standard curve which corrects for reduced-signal effects related to loss of reagent activity and linear inhibition effects. In an embodiment containing three reaction zones, the signal products generated in the second and third zones are used to construct a two-point standard curve which corrects for reduced-signal effects related loss of reagent activity and non-linear inhibition effects. The signal value from the first zone in each embodiment is plotted on the standard curve, to determine a corrected analyte concentration value.

EP 0 357 400 A2

## Description

## SELF-CORRECTED ASSAY DEVICE AND METHOD

### 1. Field of the Invention

The present invention relates to a method and device for assaying fluid-sample analytes.

### 2. References

Aouidet, A., et al., Clin Chem, 29(11):2001 (1983).
Ketterman, R., et al., J Clin Chem Clin Biochem, 22(3):245 (1984).
Kovar, K.A., et al., Clin Chem Acta, 132(3):257 (1983).
Malispina, J.P., et al., Ann Biol Clin, 38(4):207 (1980).
Moshides, J.S., J Clin Chem Clin Biochem, 25(9):583 (1987).
Sharma, A., et al., Clin Biochem, 20(3):167 (1987).

### 3. Background of the Invention

Assays for determining a variety of body-fluid analytes in a doctor's office or home setting are available. As a rule, such assays are designed for simplicity of use, on the assumption that the user has little or no training in clinical assay procedures or in reading and interpreting the results of the assay. For this reason, the assays tend to favor one-step analyte addition, with assay results being determined after a reaction end-point is reached. Analyte-addition assays of this type, while convenient for the user, generally lack accuracy and reliability. In particular, the test result may be subject to variations in reaction variables such as reagent levels and activities, particularly where enzyme reagents are involved, and temperature conditions. The accuracy and reliability of many simple assay procedures is also limited by background interference which can vary among analyte samples, and which may also be affected by the condition of the test reagents.

One type of diagnostic assay format which is generally adaptable to a one-step assay protocol is an absorptive-pad device, containing a pad or matrix designed to absorb a sample volume, and to produce an analyte-dependent chemical reaction which can be detected on the pad's surface. Examples of absorptive-pad assay devices and methods are described in U.S. Patent Nos. 3,983,005, 4,069,017, 4,144,306 and 4,447,575.

A limitation of an absorptive-pad assay format, in addition to the limitations mentioned above, is that the minimum volume of sample needed to wet the pad may contain an amount of analyte that "saturates" the detection range of the assay. In particular, where analyte detection is based on a color change detected at the surface of the reaction pad, the change in color which is detected, e.g., by reflectance absorptiometry, may be produced at a relative low analyte concentration (in contrast to the same reaction in solution, where changes in the concentration of a colored reaction product can be detected over a wide concentration range). Undiluted sample may therefore contain a higher concentration of analyte than can be effectively quantitated in a absorption-pad device.

An example of this limitation has been encountered in absorptive-pad assays for determination of serum cholesterol. This analyte, which is frequently assayed in a clinical laboratory or doctor's office, is clinically important since high blood cholesterol level, and particularly a high level of cholesterol associated with low-density lipoproteins (LDL), is directly associated with a number of serious disease conditions in humans, including coronary artery diseases, biliary obstruction, and liver or thyroid dysfunctions.

### 3. Summary of the Invention

It is therefore one general object of the invention to provide an analyte test device and method which is both simple and reliable.

Another object of the invention is to provide an apparatus using such device and employing such method to determine an analyte value which is corrected for inhibition and loss of activity effects, and which is determined from a standard curve.

Another object of the invention is to provide such a method and device for determination of analyte concentration in a fluid sample, over a broad range of analyte concentrations.

The device of the invention includes first and second reaction zones into which an analyte-containing body-fluid sample is introduced. The reaction zones are preferably porous-matrix pads designed to draw the fluid sample into the zones by surface wetting. The reactions zones may be in contact with a reservoir which receives and distributes the fluid sample to the zones.

The first reaction zone contains analyte-specific component(s) which are effective, when an analyte-containing fluid sample is added to the zone, to utilize the analyte to produce an intermediate reaction product. Signal-generating component(s) also present in the first reaction zone are effective to utilize the intermediate reaction product to produce a signal product having a measurable value which is dependent on the concentration of analyte present in the fluid sample.

The second reaction zone contains a known amount of a reference compound which is not present in the aqueous fluid sample, and compound-specific component(s) which are effective, when an analyte-containing fluid sample is added to the zone, to utilize the reference compound to produce the intermediate reaction product. Also included in this zone are the above signal-generating component(s). The two-zone device permits determination of analyte concentration based on a standard curve which is connected for background and linear-inhibition effects.

The device may further include a third reaction zone whose reaction components are like those of the second zone, but with a different known amount of reference compound. The three-zone device permits determination of analyte concentration based on a standard curve which is corrected for background and non-linear inhibition effects.

In one general embodiment, the analyte-specific reaction component(s) in the first zone, and the compound-specific component(s) in the second zone are effective to react with the analyte and reference compound, respectively, to produce $H_2O_2$. The signal-generating component(s) in this embodiment include a peroxidase and a dye or binary dye system substrate which is converted by the peroxidase, in the presence of $H_2O_2$, to a distinctively colored signal reaction product.

In one preferred embodiment of the invention in which $H_2O_2$ is generated as the intermediate reaction product, the signal-generating components further include a trapping agent effective to compete with the signal reaction product substrate, in the presence of the peroxidase enzyme, to produce a silent reaction product which is distinguishable from the signal reaction product, with utilization of $H_2O_2$. The production of the silent reaction product reduces proportionately the amount of signal reaction product generated by a given amount of analyte. The trapping agent preferably reduces the analyte-dependent amount of signal reaction product formed to between about 10%-70% of the reaction product formed in the absence of trapping agent.

In another aspect, the invention includes an apparatus for determining the amount of an analyte in an aqueous fluid sample. The apparatus includes an assay device of the type described above, preferably including structure for distributing a fluid sample to each of the reaction zones. The signal value at each zone is measured by a signal detector, typically by optical monitoring. The signal values measured in the second, or second and third zones are used to generate a single- or two-point standard curve, respectively, corrected for background and inhibition effects, and the analyte concentration value is determined from the standard curve.

Also disclosed herein is a method using the device and apparatus of the invention for determination of analyte concentration in a body-fluid sample.

These and other objects and features of the invention will becomes more fully apparent when the following detailed description of the invention is read in conjunction with the accompanying drawings.

## Brief Description of the Drawings

Figure 1 shows a strip-type assay device constructed according to one embodiment of the invention;

Figure 2 shows an enlarged sectional view of the Figure 1 device, taken along line 2-2 in Figure 1;

Figure 3 illustrates a typical color-intensity result achieved with the strip shown in Figure 1;

Figure 4 shows an assay device for use in qualitative analyte determination, in accordance with another embodiment of the invention;

Figure 5 shows a signal/concentration plot used in calculating analyte concentration from a single-point standard curve, in accordance with the method of the invention;

Fig. 6 shows a signal/concentration plot used in calculating analyte concentration from a two-point standard curve in accordance with another embodiment of the method of the invention;

Fig. 7 illustrates the use of a four-zone assay method for determination of total serum cholesterol;

Fig. 8 illustrates the use of a four-zone assay method for determination of total and free serum cholesterol;

Fig. 9 shows general reaction components and products generated in an embodiment of the invention containing a trapping agent;

Fig. 10 shows the reaction scheme for determination of serum cholesterol in a device containing the components shown in Figure 9;

Fig. 11 shows plots of measured reflectance as a function of cholesterol concentration, in the absence (solid rectangles) and presence (open rectangles) of a trapping agent;

Fig. 12 shows the reaction of two dye components in the presence of $H_2O_2$ and peroxidase to produce a colored reaction product;

Fig. 13 illustrates a trapping reaction which competes with the Figure 12 reaction, in the presence of a trapping agent, to produce a colorless reaction product;

Figure 14 is a schematic view of an assay apparatus constructed according to the present invention; and

Figure 15 is a flow diagram of operations performed by the processor in the Figure 14 apparatus, for determining total analyte concentration using the single-point and two-point standard curve methods of the invention.

## Detailed Description of the Invention

### A. Assay Device

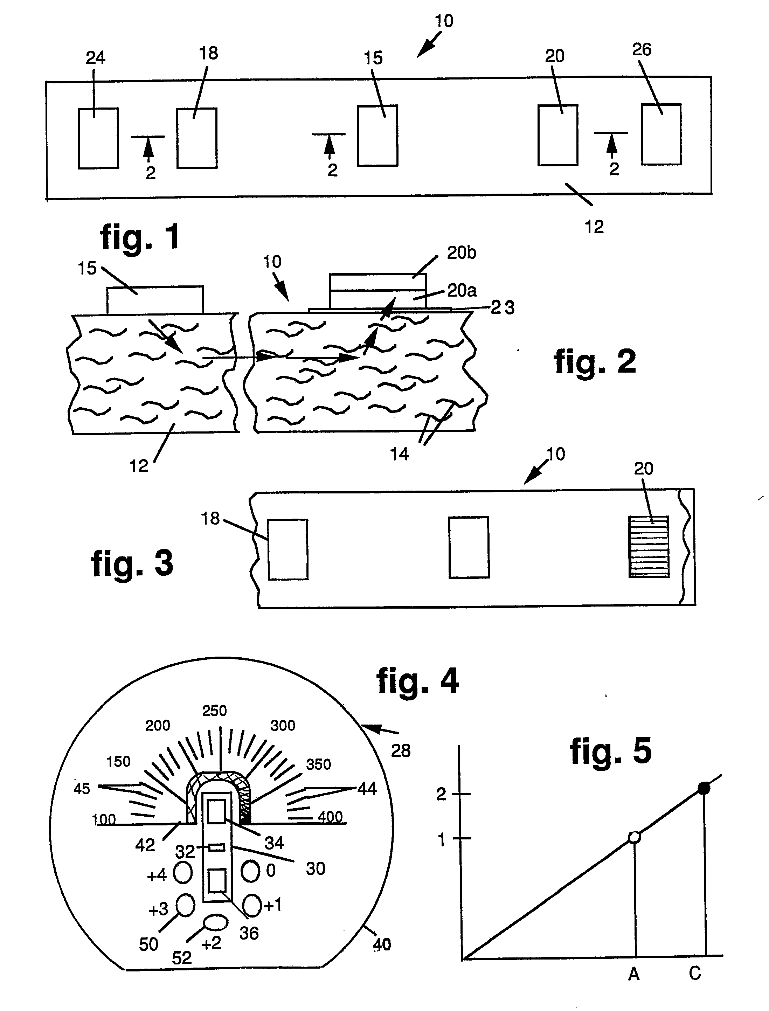

Figure 1 shows an assay device 10 constructed according to one embodiment of the invention. The device includes a porous- matrix strip 12 formed of a network of fibers, such as fibers 14 seen in Figure 2. The porous, fibrous network construction is designed to draw fluid applied to the strip by surface wetting through the strip. That is, a fluid sample applied to one zone of the strip will migrate by surface wetting toward opposite ends of the strip until the entire strip is wetted. The fluid sample is applied to a sample pad 15 located at the center of the strip. The pad and the strip are preferably constructed to partially remove unwanted particles, such as blood cells, as the sample fluid migrates through the pad toward the strip.

A variety of fibrous strip materials, such as are used in fibrous-mat filters, including cellulose, cellulose acetate, and glass fibrous matrices, are suitable materials for the strip and sample pad. The fibers may be crosslinked, if desired, by chemical crosslinking, heat fusion, or the like. Also suitable are porous substrates, such as scintered glass, fused polymer beads, and the like whose wettability and dimension of interstices are such as to promote movement of an aqueous medium into the matrix by surface wetting. One suitable material for the pad and strip is glass fiber filter material which acts to separate particles from non-particulate solute material in a fluid sample on the basis of particle size. Thus, for example, a blood sample applied to the pad is drawn through the strip away from the pad, with the concentration of blood particles in the sample decreasing at the leading edge of the migrating sample.

The strip has typical dimensions of about 1-5 cm in length, 4-10 mm in width, and 50-500 microns in thickness. The pad has about the same side dimensions as the width of the strip, and has a thickness between about 5-500 microns.

Attached to the strip are two reaction pads 18, 20 which are also referred to herein as first and second reaction zones, respectively. These reaction zones are formed of a fibrous matrix filter material, such as described above, designed to draw aqueous fluid by surface wetting. As seen in Figure 2, pad 20, which is representative, is composed of an inner and outer layers, such as layers 20a and 20b, respectively. The layers in the pad are attached to one another by a conventional fluid-permeable adhesive or the like.

With continued reference to Figure 2, the pads in the device are attached to and separated from the upper surface of strip 12 by a membrane, such as membrane 23, which is permeable to selected fluid components in the sample fluid, but impermeable to particles or larger solutes in the sample. The size exclusion limit of the membrane is selected to allow passage of desired sample components into the pads, and block undesired material. For example, in an assay device for determination of total serum cholesterol in a blood sample, the size exclusion limit of the membrane is selected to allow passage of lipoprotein particles, such as HDL and LDL particles, but exclude blood cells, such as red blood cells.

The membrane is preferably light-colored and opaque, to mask the color effect of filtered red blood cells or the like in the strip below the pad. The pads are attached to the associated membranes, and the membranes are attached to the upper surface of the strip by a conventional fluid-permeable adhesive or the like.

In an assay procedure, a fluid sample introduced on pad 15 at the center of the strip is drawn by surface wetting into strip 12, and from the center of the strip outwardly through associated membranes into two reaction zones, with the concentration of analyte in each reaction zone being essentially the same, since each pad is saturated with the analyte sample fluid. Strip 12 and pad 15 thus serve as a reservoir which provides means for distributing a fluid sample to each of the reaction zones at substantially the same analyte concentration in each zone.

The first reaction zone, i.e., zone 18 in device 10, contains one or more components which are effective to react specifically with the analyte to generate an intermediate reaction product whose concentration is dependent on the concentration of analyte in the zone. The component or components, which are also referred to herein as analyte-specific component means, typically include an analyte-specific enzyme which can react specifically with the analyte to generate the intermediate product or, alternatively, to generate an analyte-related product which can serve as a substrate for a second reaction leading to the intermediate product.

Also included in the first reaction zone are signal-generating component(s) which are effective to utilize the above intermediate reaction product to produce a signal product having a measurable value which is dependent on the concentration of intermediate product in the reaction zone. The component or components necessary to utilize the intermediate product to produce the desired signal product are also referred to herein as signal-generating component means.

The second reaction zone, i.e., pad 20 in device 10, contains a known amount of a reference compound which is not present in the aqueous fluid sample to be assayed, and which is preferably freely soluble in the sample fluid introduced into the zone. The second zone also contains compound-specific component(s) which are effective, when an analyte-containing fluid sample is added to the zone, to utilize the reference compound to produce the above intermediate reaction product. The compound-specific reaction component(s) are also referred to herein compound-specific component means.

In the specific construction shown in Figures 1-3, the reference compound and the compound-specific component(s) are contained in different layers 20a and 20b of pad 20, typically with the reference compound being in the lower layer. Thus, as fluid sample is drawn into the pad, the reference compound is dissolved in the sample fluid and carried into the upper layer, where it reacts with the compound-specific components.

Also included in the second reaction zone are the above signal-generating component(s) which are effective to utilize the intermediate reaction product formed by reaction with the reference compound to produce a signal reaction product. The signal-generating components, or component means, are preferably contained in the same dry-layer compartment as the compound-specific-specific component(s).

Exemplary analyte specific component(s), signal-generating component(s), compound-specific component(s), and reference compound are given in Section B below. The reaction pads may also contain a dye-trapping agent, as described in Section E below.

The device shown in Figure 1 contains two additional reaction pads 24, 26 which provide third and fourth reaction zones, respectively. In one preferred embodiment of the invention, the third reaction zone, i.e., pad 24, includes the same components as the second reaction zone, but a different known amount of reference compound. The amounts of reference compound in the second and third zones are such as to produce different detectable signal product levels, when sample fluid is introduced into the zones, and each at non-saturating signal product levels.

The fourth reaction zone may serve one of a variety of purposes in the analyte assay. In one device, the fourth reaction zone is a duplicate of the first reaction zone, and thus provides a check on the reproducibility and of the assay reaction.

In another embodiment, the fourth reaction zone may contain different analyte-specific component(s) for determining a different analyte, but with the generation of the same intermediate reaction product. As one example, the first reaction zones may contain cholesterol oxidase, for determination of free cholesterol, and the fourth zone, glucose oxidase, for determination of glucose, with both reactions generating the intermediate reaction product $H_2O_2$.

As another example, the first reaction zones may contain cholesterol esterase and cholesterol oxidase, for determination of total serum cholesterol, as detailed below, the fourth zone, cholesterol oxidase only, for determination of free cholesterol, as described below in Section B.

Figure 4 illustrates an embodiment of an assay device 28 constructed according to another general embodiment of the invention, for use in a qualitative, self-corrected analyte assay. Device 28 is composed of a porous matrix strip 30, similar to above strip 12, a fluid-sample pad 32, similar to above pad 15, and first and second reaction pads or zones 34, 36, having a two-layer construction similar to pads 18, 20, respectively.

The first pad or reaction zone contains the analyte- specific component(s) or component means and signal-generating component(s), or component means, described above with reference to pad 18 in device 10. The first reaction pad is designed to utilize analyte to produce an intermediate product, such as $H_2O_2$, with subsequent reaction to produce the desired signal product.

The second pad or reaction zone contains a reference compound, compound-specific component(s) or component means and signal-generating component(s), or component means, described above with reference to pad 20 in device 10. The second reaction pad is thus designed to utilize reference compound in the pad to produce an intermediate product, such as $H_2O_2$, and utilize the intermediate reaction product to produce the desired signal product.

The strip and attached pads are carried on a plastic card 40 or the like having a variety of indicia and color-intensity markers for use in qualitative determination of analyte concentration. In the device shown, pad 34 is bordered by a color-intensity spectrum band 42 whose increasing color intensity corresponds to increasing analyte concentration values in a typical expected range of values. For example, in a Figure 4 device, which is intended for cholesterol determination, the color band has an intensity spectrum corresponding to 100-400 mg/dl cholesterol. A plurality of indicia markings, such as marking 44, indicate the corresponding analyte concentration, as indicated. A outer row of dots, such as dots 45, corresponds to 5% increments in concentration values. Thus, the dots immediately to right of the "100," "200," and "300" concentration-value indicia correspond to concentration values of "105", "210", and "315", respectively. The purpose of the dots, for correcting the analyte value determined from the spectrum band 42, will be described below in Section C.

The second reaction zone in the device is surrounded by a plurality of color-intensity markers, such as markers 50, 52, each corresponding to a reference correction value ranging from 0 to +4. The color intensity of the 0-correction color marker is the expected color intensity which is produced under optimal reaction conditions, i.e., without reduction in color intensity due to loss of activity of one or more of the reagents active in color development, or inhibition of the color-generating reactions by the fluid sample added to the pad.

Each successively lower intensity marker corresponds to the color intensity expected with increasing degrees in inhibition and/or loss of enzyme activity. In the embodiment illustrated, each intensity markers corresponds to a decrement in color intensity corresponding roughly to 5%, or a total of 20% inhibition at the +4 marker.

B. Assay Component(s)

The analyte-specific component(s) employed in the first reaction zone typically include an analyte-specific enzyme which is effective to react specifically with the analyte, or an analyte-derived product, and in some cases, analogs of the analyte or analyte-derived products, to generate the above intermediate product. The components in the analyte-specific component means include all of the enzyme, cofactor, and substrate components (other than the analyte itself) necessary to produce the intermediate reaction product in the presence of the analyte.

A wide range of enzymes or enzyme systems which act on selected body-fluid analytes to produce suitable intermediate reaction products are known. Table I shows several exemplary analytes for which suitable analyte-specific oxidases exist. As seen, the analytes may themselves be the substrate of the analyte-specific enzyme, as in the case of glucose, uric acid, amino acid oxidase, and free (non-esterified) cholesterol. Here the analyte-specific oxidase reagents may include only the oxidase enzyme.

Alternatively, the analyte may be first converted by primary analyte-specific enzyme(s) to produce the substrate recognized by the oxidase enzyme. Here the analyte-specific oxidase reagents include both the oxidase and additional enzyme for converting the analyte to the oxidase substrate.

In the case of esterified cholesterol, for example, the analyte-specific oxidase reagents include cholesterol esterase, for converting cholesterol in esterified form to free cholesterol, and cholesterol oxidase, which produces cholestenone and $H_2O_2$ in the presence of oxygen.

The analyte-specific oxidase reagents for determination of serum triglyceride include lipase, which hydrolyses triglyceride to glycerol and free fatty acids; glycerol kinase, which converts glycerol to glycerol-phosphate in the presence of ATP; and glycerol-phosphate oxidase, which reacts with glycerol-phosphate to produce dihydroxyacetonephosphate plus $H_2O_2$.

The analyte-specific oxidase reagents for determination of creatinine include creatinine amidohydrolase, which converts creatinine to urea and sarcosine, and sarcosine oxidase, which converts sarcosine to glycine and formaldehyde, with production of $H_2O_2$.

TABLE I

| Analyte | Substrate | Oxidase |
|---------|-----------|---------|
| glucose | glucose | glucose oxidase |
| uric acid | uric acid | uricase |
| amino acid | amino acid | amino acid oxidase |
| free cholesterol | cholesterol | cholesterol oxidase |
| esterified cholesterol | cholesterol | cholesterol oxidase |
| triglyceride | L-glycerol phosphate | L-glycerol phophate oxidase |
| creatinine | sarcosine | sarcosine oxidase |

It will be appreciated that a variety of other analytes may be assayed by suitable selection of an enzyme or enzyme system capable of reacting with the analyte, with the downstream production of $H_2O_2$ by a suitable substrate-specific oxidase. Alternatively, the analyte may be an oxidase enzyme capable of reacting with a suitable substrate in the oxidase, where the analyte-specific oxidase reagents now include the oxidase-specific substrate, rather than the oxidase enzyme itself.

Another class of enzymes which are suitable for use in the present invention are the dehydrogenases, such as hydroxyacyl dehydrogenase, and various Krebs-cycle dehydrogenases, where the intermediate reaction product is reduced NAD, NADH, or FAD. Since reduced nucleotide cofactors, such as NADH, may be present in the blood-fluid sample, it may be necessary to remove the co-factors prior to introducing the sample into the reaction zone(s). Where the device includes a sample reservoir strip, as described in Section A, the reduced cofactor may be removed catalytically or enzymatically by including suitable immobilized cofactor oxidizing reagents in the pad and/or strip through which the sample migrates, before entering the reaction zone.

A variety of other enzyme systems capable of converting an analyte to an intermediate reaction product which is not present in the analyte-containing fluid sample and which can be converted to a detectable signal product are suitable.

The signal-generating reaction component(s) in the reaction zones typically include one or more enzymes which react specifically with and/or utilize the intermediate reaction product to produce a detectable reaction product. One preferred signal-generating component means suitable for an oxidase enzyme system which produces $H_2O_2$ as an intermediate reaction product includes a peroxidase, and a dye which is converted by the peroxidase, in the presence of $H_2O_2$, to a distinctively colored, signal reaction product.

The peroxidase enzyme is a hydrogen-peroxide oxidoreductase, such as horseradish peroxidase, myeloperoxidase, and lactoperoxidase, which catalyses the reaction:

Donor $+ H_2O_2 \rightarrow$ oxidized donor $+ 2H_2O_2$.

The specificity of the enzyme for the donor is generally low, and a number of phenols, aminophenols, diamines, and indolephenols are active. In the present invention, the donor, or substrate reagent, is selected among a variety of known compounds or pairs of compounds which undergo reaction to a detectable, typically chromogenic reaction product as a result of peroxidase-catalysed oxidation.

Exemplary single donor compounds include 0-pheylenediamine, amidopyrine (Aouidet), and naphthalene-2,3-dicarboxaldehyde (Malaspina). Typically formation of a colored reaction product involves dimer formation. Examples of donor compound pairs which are suitable include the following primary/secondary compound pairs: 4-aminoantipyrine (4AAP)/2-hydroxy-3,5-dichlorobenzenesulfonate (Sharma), which form a red quinoneimine chromophoric compound with an absorption max at 510 nm; 4AAP/phenol (Ketterman);

6

4AAP/2,4,6-tribromo-3-hydroxybenzoic acid, which forms quinoneimine dye with an absorption max at 515 nm (Moshides); 4AAP/p-hydroxybenzoate, and 3-methylbenzothiazolin-2-one hydrazone/3-dimethylaminobenzoic acid, which forms a compound with absorption max at 590 nm (Kovar).

For quantitative determination of analyte, it is, of course, important that the level of signal reaction product which is produced be proportional to initial analyte concentration. This requires first, that the amount of $H_2O_2$ available for formation of signal product be dependent on analyte concentration in the reaction mixture, and secondly, that the amount of reaction product generated also be dependent on the amount of $H_2O_2$ formed. These requirements can be met by employing amounts of analyte-specific oxidase reagents, peroxidase, and substrate reagent which are essentially rate limited only by the amount of analyte introduced initially into the reaction pad. Suitable concentrations of the reagents for use in various oxidase-containing assay systems have been reported (see references cited above). The concentrations of the reagents employed in the serum cholesterol assay device described in the examples below are typical.

Other signal-generating component means are suitable. For example, where the intermediate reaction product is a reduced nucleotide cofactor, the signal-generating components may include a suitable reductase enzyme, such as diaphorase, and a dye which is converted to a reduced form with a distinctive color change.

As indicated above, the reference compound and compound-specific component(s) are selected to produce a predetermined level of signal reaction product, via the intermediate reaction product. The selection of the reference compound is guided by the following requirements: The compound is not present in measurable amounts in the fluid sample introduced into the reference-compound pad. Secondly, the reference compound must be capable of reaction with suitable compound-specific reaction means, with the production of the desired intermediate reaction product. In addition, the reference compound is preferably readily soluble in water, or easily solubilized by detergents or the like which can be included in the reaction zone with interference with the signal-product generating reactions occurring in the zone. One preferred reference compound, in the general embodiment where the intermediate reaction product is $H_2O_2$, is a D-amino acid, such as D-glycine, D-proline or D-alanine. The compound-specific oxidase component in this embodiment is D-amino acid oxidase, which is specific to D-amino acids.

The assay components just described are preferably included in the associated reaction pad by adding a known volume of assay-component solution to the pad, then dehydrating, e.g., by lyophilization. In the case of the reference-compound pad, a known amount of a solution of the reference compound is introduced into one layer, and a solution of the compound-specific and signal-generating components are introduced into the second layer. After dehydration, the two layers are joined as described above.

One exemplary device, described in Examples 1 and 2, is designed for determination of total serum cholesterol. The analyte-specific reaction components in the first reaction zone include cholesterol ester hydrolase, for releasing cholesterol in free (non-esterified) form from serum lipo proteins, and cholesterol oxidase, for converting free cholesterol to cholestenone and $H_2O_2$. The signal-generating components in the first zone include a peroxidase, and a dye or dyes (see Example 1 below) which changes from clear to colored with peroxidase-catalyzed oxidation in the presence of $H_2O_2$.

The second reaction zone includes a known amount of D-amino acid, the corresponding amino acid oxidase and the above signal-generating components for generating the signal product in the presence of $H_2O_2$. Preferred concentrations of the reagents and reference compound are given in Example 1 below.

One exemplary three-zone device, designed for determination of total serum cholesterol, is identical to the device just described, but contains a third reaction zone with a different, predetermined amount of D-amino acid. A fourth, optional reaction zone in the device contains the same assay components as the first reaction zone, to provide a measure of signal level reproducibility.

Table I below shows the components present in the three reaction zones, and the reactions occurring in each zone:

Table I

| Components | Zone | | |
|---|---|---|---|
| | 1 | 2 | 3 |
| Sample (cholesterol esters + free cholesterol) | + | + | + |
| cholesterol esterase | + | | |
| cholesterol oxidase | | + | |
| D-amino acid | | + | + |
| D-amino acid oxidase | | + | + |
| $H_2O_2$ generated $H_2O_2$ | + | + | + |
| peroxidase | + | + | + |
| + dye(s) | + | + | + |
| colored dye | + | + | + |

The test devices just described are designed for assaying total serum cholesterol, that is, cholesterol which is associated with lipoproteins in free (non-esterified form) and cholesterol esters which are associated with lipoproteins. The device may be modified for assaying free cholesterol only, and/or cholesterol esters, by modifying the fourth reaction zone to react with free, but not esterified cholesterol. In this device, the first and fourth reaction zones are identical, except that the fourth zone is lacking in cholesterol esterase. The composition of the four reaction regions is given in Table II below. The manipulation of the four signal values to calculate corrected values of total, free and esterified cholesterol is described in Section C below.

Table II

| Components | Zone | | | |
|---|---|---|---|---|
| | 1 | 2 | 3 | 4 |
| Sample (cholesterol esters free cholesterol) | + | + | + | + |
| cholesterol esterase | + | | | |
| cholesterol oxidase | | + | | + |
| D-amino acid | | + | + | |
| D-amino acid oxidase | | + | + | |
| generated $H_2O_2$ + + | | + | + | |
| peroxidase | + | + | + | + |
| dye | + | + | + | + |
| colored dye | + | + | + | + |

## C. Two-Zone Self-Corrected Assay Method

The method described in this section uses a two-zone assay device in a self-corrected assay method. The analyte determination made according to this method may be either qualitative, based on the color intensities of the two zones determined visually by the user, or quantitative, based on a quantitative signal value determination made by conventional reflectance intensity measurements or the like, as described in Section F below.

8

Describing first a two-zone assay using the device illustrated in Figures 1-3, a body-fluid sample, such as a whole blood sample, is applied to pad 15, from which the sample is drawn from the strip into the two reaction zones. In the case of a blood sample, particulate material in the sample, such as blood cells, is filtered, and only cell-free serum is drawn into the pads.

Figure 3 illustrates a typical reaction result, where the different levels of shading in pads, 18 and 20 represent different final levels of signal reaction, after the pad reactions have gone substantially to completion. The amount of signal product in each pad may be determined quantitatively by a number of known optical methods, such as optical-beam reflectivity, as described below, and may involve time-dependent kinetic measurements rather than as end-point measurements.

The measured values are used to calculate a self-corrected analyte concentration, using the steps outlined below and illustrated in the plot in Figure 5. The reference-compound signal level measured in the second reaction zone, indicated at 2 on the ordinate in the Figure 5 plot, corresponds to a known concentration of analyte, indicated at C along the abscissa in the figure. The singlereference-compound value is used to establish a single-point standard curve extending from the origin of the plot, as shown.

A corrected analyte value is now determined by plotting the measured signal value from the first reaction zone on the standard curve, and reading the corresponding analyte concentration on the plot, indicated at A in Figure 5. It will be appreciated that the analyte concentration is self-corrected for reduced signal effects due either to inhibition of the common signal-generating components in the two reaction zones, and/or loss of enzyme activity in this system. Inhibition effects represented by the curve are linear effects, in that the standard curve is a linear plot extending through the origin of signal value/concentration axes.

More generally, the reduction in signal value observed in the second reaction zone will also apply to that observed in the first reaction zone to the extent that:

(a) sample inhibition or loss of activity effects are confined to the common signal-generating component(s) in each reaction pad, or

(b) any sample-inhibition or loss of activity effects which apply to the signal-generating component(s) apply equally to the analyte-specific components in the first reaction zone and the compound-specific component(s) in the second reaction zone.

A method for qualitative determination of analyte concentration using the two-zone device described with respect to Figure 4 will now be considered. As above, a body fluid sample is applied to the center sample pad from which it migrates into first and second zones 34 and 36, respectively. After a given reaction period, preferably at the reaction end point, the signal value--in this case, color intensity-- is matched with the a color intensity region of the intensity spectrum band 42, to determine an uncorrected analyte concentration, expressed for example, in mg analyte/dl sample fluid.

At the same time, uptake of fluid sample into the second pad triggers a signal reaction product reaction whose final color intensity is dependent on the amount of reference compound in the pad, and which may be reduced by sample-inhibition effects and/or loss of reagent activity. As noted in Section A above, the percent inhibition or reduction of the signal level can be qualitatively assigned a color-intensity value from 0 to +4, corresponding to an decrease in maximum expected signal value from 0 to 20%, respectively. This percent reduction in color intensity will approximate the percent reduction observed in the first zone, subject to the factors discussed above.

After determining uncorrected the analyte concentration from the first zone reading, the assay value is corrected by (a) locating the dot on the outer ring of the concentration indicia closest to the uncorrected value indicia, (b) advancing 0-4 dots to the right, corresponding to the correction value determined from the second reaction zone, and (c) determining the concentration closest to the final dot advanced to. This procedure has the effect of adding to the uncorrected analyte value, a percent increase which approximately offsets the percent of inhibition observed from the second reaction zone.

By way of illustration, assume that an uncorrected analyte concentration value of "270" and an inhibition level of "+3" are determined initially from the two reaction zones. The user than selects the dot closest to the "270" indicium, advances, three dots to the right, and selects the indicium closest to the last dot--given a corrected analyte concentration value in this case of "310". This is the value that would have been measured, approximately, in the absence of inhibition effects.

### D. Three-Zone Self-Corrected Assay Method

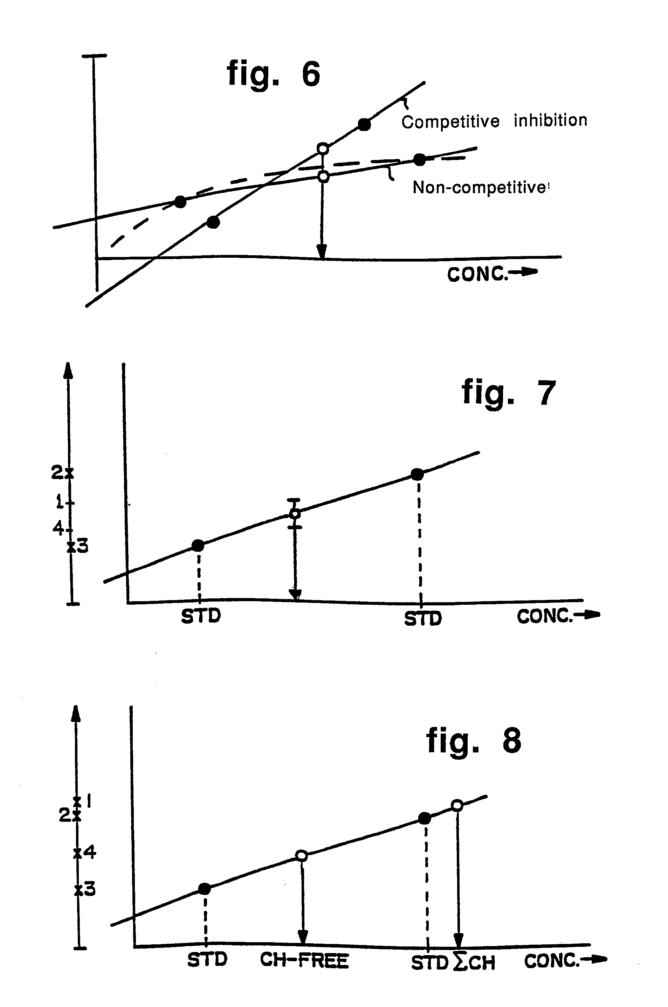

A second general embodiment of the assay method of the invention is based on a standard curve generated by two reference value determinations. The method uses the assay device described above in which second and third reaction zones produce two distinct positive reference values when sample fluid is added to the device. As will be seen with reference to Figure 6 below, this embodiment of the method corrects not only for background signal level, but also compensates for non-linear competitive and noncompetitive enzyme inhibition effects.

Figure 6 shows plots of two-point competitive and non-competitive inhibition curves, where each plot is constructed by drawing a straight line through the two reference-value points (solid circles) determined from the second and third reaction zones in the assay device. As seen, competitive inhibition is characterized by decreasing inhibition at greater substrate concentrations, and a negative intercept at the zero-substrate concentration, reflecting the level of inhibition at zero-substrate concentration.

Non-competitive inhibition, by contrast, is characterized by a zero intercept at the zero-substrate concentration, and a progressively greater inhibition at higher substrate concentrations. Although it is possible, using standard curve-fitting methods, to construct the non-linear plot shown at dashed lines in the figure, the curve can be approximated, in the zone between the two reference-value points (solid circles), by a straight line plot drawn between the points.

The first zone gives an analyte concentration value which is uncorrected for background or competitive inhibition. This value is plotted on standard two-point curve (open circle) and the corresponding analyte concentration is read from the intercept with the abscissa (concentration).

According to an important feature of this method, the analyte value determined from the intercept is corrected for both background and enzyme inhibition effects. The background correction is inherent from the fact that first, the same signal value attributable to background is present (or assumed to be present) in each of the three readings, and second, that the two reference points are plotted according to known concentrations of reference compound, and are thus both corrected for background effects. The effect of non-competitive inhibition is corrected inherently by the two-point plot, assuming the same degree of non-competitive inhibition is present in each reaction zone.

A first exemplary method, for determination of total serum cholesterol, will be described with respect to signal value plot shown in Figure 7. The device used in the method has the four-zone construction shown in Figure 1, where the first three zones contain the reaction components shown in Table I, and the fourth, the same components as the first zone. That is, the first and fourth zones are designed to measure total serum cholesterol in duplicate, and the second and third zones are designed to provide two different reference point signal values.

The method is carried out as above, by adding a blood sample to the central pad, and allowing the filtered serum sample to be drawn up into each of the four reaction zones. At intervals during the reaction, and/or when the reaction in each pad has run substantially to completion, the color intensity of each pad is measured, e.g., by optical-beam reflectivity measurement.

Measured signal values for each of the four zones are shown at the line to the left in Figure 7. The second and third measurements, which represent different amounts of reference compound corresponding to different preselected cholesterol concentrations, indicated at "STD" in the figure, are used to generate the two-point standard curve shown in the figure.

The first and fourth signal measurements, which represent duplicates of the total cholesterol value, are averaged, and the average value (represented by an open circle) is plotted on the standard curve, and the corresponding cholesterol concentration is read from the curve.

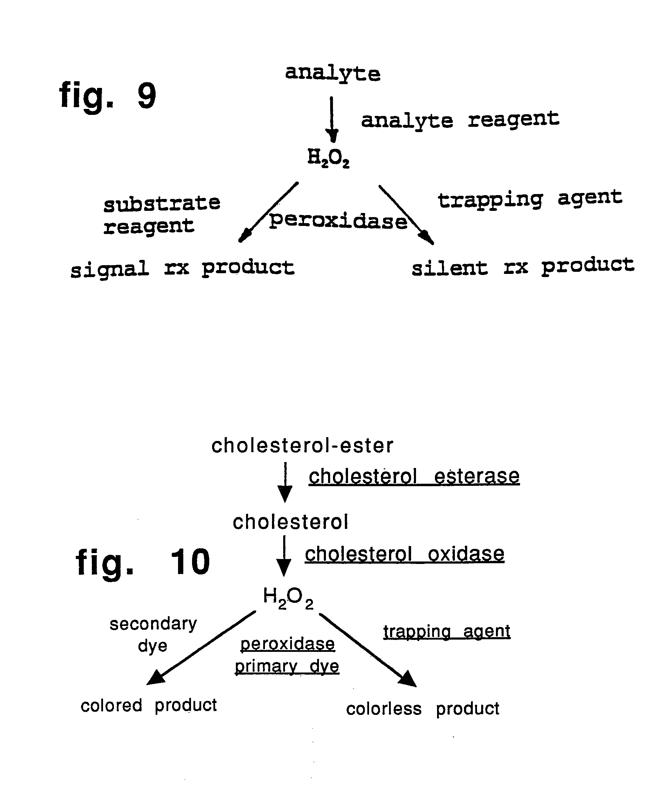

A second exemplary method, for determination of free and total serum cholesterol, will be described with respect to signal value plot shown in Figure 8. The device used in the method has the four-zone construction shown in Figure 1, and the four reaction zones contain the reaction components shown in Table II above. The method is carried out as above.

Measured signal values for each of the four zones are shown at the line to the left in Figure 8. The second and third measurements are used, as above, to construct standard plot shown in the figure. The first and fourth reaction zones, which correspond to the signal values produced for total cholesterol (first zone) and free cholesterol only (fourth zone) are then determined by plotting each value on the standard curve, as shown.

In both of the examples above, the three-zone assay method gives an analyte value which is (a) determined from a standard curve and (b) corrected for competitive or non-competitive inhibition effects, and for reduction in signal activity due to loss of reagent activity in the reaction pads. In addition the first method described provides a measure of the reliability of the assay.

## E. Trapping Agent

As discussed above, one of the limitations of prior art reaction-pad assay systems is the limited range of analyte concentrations which can be assayed, due to color saturation in the pad at relatively low analyte concentrations.

This problem has been solved, in one embodiment of the present invention, by including a trapping agent in the signal-reaction components. This trapping agent is effective to compete with the substrate of the signal reaction product, i.e., the dye or binary dye system, to produce a silent reaction product which is distinguishable from the signal reaction product, with utilization of $H_2O_2$, as illustrated in Figure 9. Typically, where the signal product has a relatively high absorption coefficient in the visible light range, the silent reaction product is a colorless or weakly colored product.

The trapping agent thus proportionately reduces the amount of signal reaction product generated by a given quantity of analyte, i.e., the level of signal reaction product which is detected at a given wavelength. Preferably the amount of trapping agent is such as to reduce the amount of signal reaction product formed to between about 10%-70% of the amount formed in the absence of the trapping agent.

The general principles of the trapping reaction are illustrated in Figure 10, which shows reaction components and products formed in the first reaction zone in an assay device for detection of serum cholesterol. The analytespecific components (cholesterol esterase and cholesterol oxidase) and the signal-generating components (peroxidase, primary and secondary dyes in a binary dye system, and the trapping agent) in the reaction zone are indicated by underlining. The same signal-generating components are included in the reference-compound reaction zones.

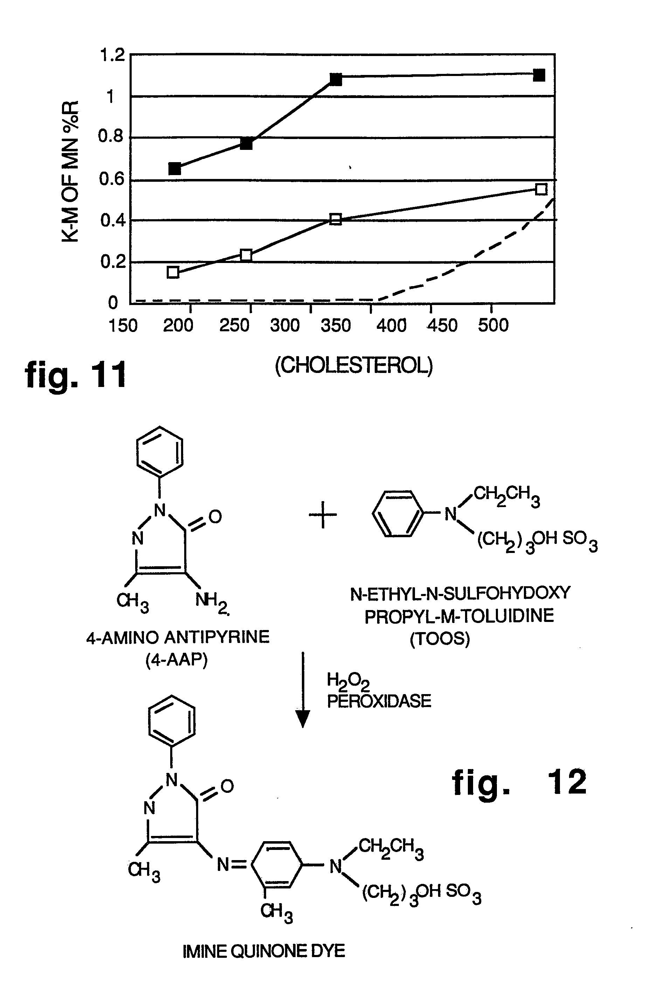

The analyte-dependent reduction in signal product in the Figure 10 reaction scheme is illustrated in Figure 11, which shows plots of measured signal product formation as a function of serum cholesterol, both with (open rectangles) or without (closed rectangles) trapping agent in the first reaction zone. As seen in the figure, color saturation (measured by reflectance at 550 nm) occurs at a reflectance value of about 1.1. In the absence of trapping agent, this value is reached with an initial serum cholesterol of between 300-350 mg/dl, which is thus the highest cholesterol concentrations which can be quantitated on the basis of detectable changes in signal product.

In the system containing trapping agent, the amount of signal product produced is at all points proportional to analyte concentration, and generally 60-80% lower than in the absence of trapping agent. The plot demonstrates that a severalfold wider range of cholesterol can be quantitated according to the present invention.

It will be appreciated that the desired analyte-dependent reduction in signal reaction product requires that the consumption of intermediate reaction product in the trapping pathway in fact be competitive with the rate of formation of signal reaction product. If the rate of consumption of intermediate reaction product in the trapping pathway is too slow, little or no reduction in signal product will be observed. On the other hand, if the rate of consumption of intermediate reaction product in the trapping pathway is too rapid, little or no signal product will be produced, at least until the all of the trapping agent is consumed.

An illustration of a system in which the intermediate reaction product is consumed too rapidly by quenching is one in which $H_2O_2$ is rapidly broken down by ascorbic acid used as a quenching agent. Experiments conducted in support of the present invention indicate that $H_2O_2$ breakdown is so rapid that essentially no signal product forms until the ascorbic acid is depleted. The theoretical behavior of this system is illustrated by the dashed line in Figure 11. The formation of signal product here is essentially suppressed up to an analyte concentration at which the quenching agent is depleted by $H_2O_2$. Above this analyte concentration, the reaction curve resembles the upper curve in the figure, where signal product is generated in the absence of trapping agent. Similar results have been observed when catalase is used to quench $H_2O_2$ in a reaction pad.

The limitations of a rapid quenching mechanism then are first that analyte is not detected below a certain threshold level, and secondly, that the range of analyte concentrations which can be detected is still relatively narrow, although shifted toward higher values.

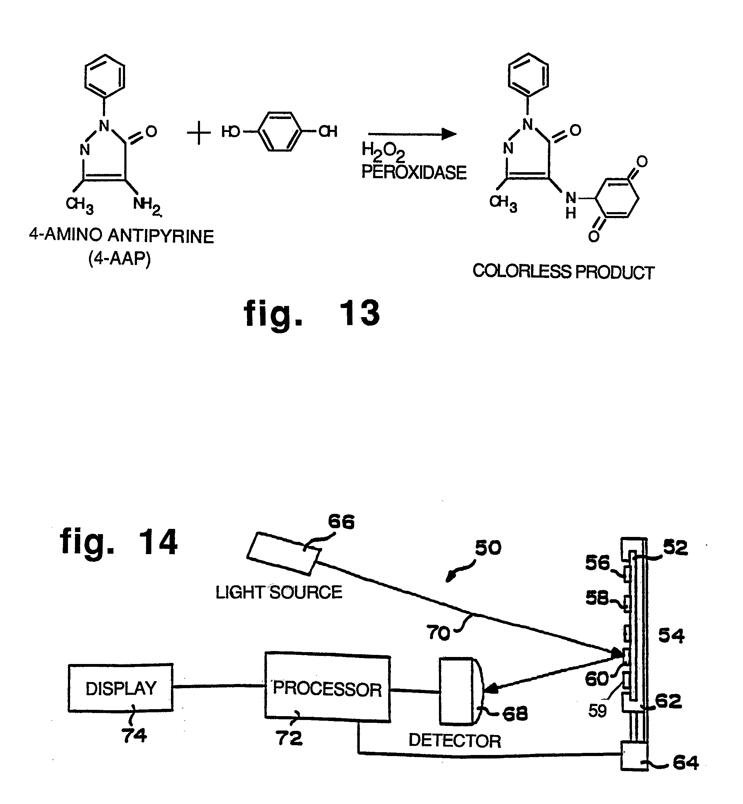

One exemplary reaction system, according to the present invention, is illustrated in Figures 12 and 13. The dye substrate here is a binary dye system which includes a primary substrate, 4-amino antipyrine (4AAP) and a secondary substrate, N-ethyl-N-sulfohydroxy propyl-m-toluidine (TOOS). The peroxidase-catalyzed reaction, in the presence of $H_2O_2$, produces a purple imine quinone dye having the structure seen at the bottom in Figure 12. The compound has a strong absorption at about 550 nm.

Figure 13 shows the reaction produced by a benzenediol trapping dye (1,4,-benzenediol or hydroquinone) in the same reaction system. As seen, the trapping dye here competes with TOOS for reaction with 4AAP, forming an essentially non-chromogenic product shown at the bottom in the figure. The non-chromogenic property of the product is likely due to the ring quinone oxygen groups which are sufficiently electron withdrawing to reduce the double bond character of the ring-carbon/nitrogen bond, such that the resonance structure in the compound necessary for absorption in the visible light range is effectively lost.

Factors which govern the extent to which the trapping dye reduces the amount of signal product formed include the relative concentrations of TOOS and hydroxyquinone, and the relative reactivities of the two compounds with 4APP in the presence of $H_2O_2$ and peroxidase. In one exemplary system, the substrate reagents include 4AAP and TOOS, and the trapping agent is 1,4-benzenediol, at a concentration of about between about 0.5 to 2 mM, and preferably about 1 mM.

Other trapping agents which may be employed in this system include a variety of substituted and benzenediols and related compounds capable of reacting with 4AAP in the presence of $H_2O_2$ and peroxidase, to form a non-colored or weakly colored compound. In particular, compounds capable of reacting with 4AAP or derivatives thereof which have electron withdrawing substituents capable of effectively eliminating resonance between the rings in the product, as indicated above, are suitable. Exemplary agents include 1,3-benzenediol and 1,4,-benezenediol.

Alternatively, in where the two-substrate system above, the trapping agent may compete with the primary substrate for reaction with TOOS, to reduce signal product formation.

In another general embodiment, the substrate reagent is a single compound which typically undergoes peroxidase-catalyzed dimer formation to form a signal reaction product, the trapping agent competes with the dimer reaction, with formation of a silent product formed by coupling the trapping agent to the substrate reagent. Here the trapping agent may be any of a number of substrates which react with the single compound to produce a silent reaction product. As an example, where 0-phenylenediamine is employed as a single compound substrate reagent, the trapping agent may be a benezenediol, such as hydroquinone.

## F. Assays Apparatus

In another aspect, the invention includes an apparatus for determining the amount of an analyte in an aqueous fluid sample. Figure 14 shows a simplified, schematic view of an apparatus 50 constructed in accordance with the invention.

The apparatus includes an assay device 52 which is similar to that described in Figure 1, and which includes a strip 54 and first, second, third and fourth reaction zones 56, 58, 59, and 60, respectively. Also included in the apparatus is a device holder 62 which is slidable, under the control of an actuator 64, to place the reaction zones at a measurement position occupied by zone 60 in the figure.

In the apparatus illustrated, the level of signal product is measured by reflectance spectroscopy. Means for measuring the signal value in this embodiment includes a light source 66 and a reflected-light detector 68. Source 66, which directs a focused selected-wavelength light beam 70 at the measurement position may be a low-power laser, a monochromatic, non-coherent light source whose beam is focused by a suitable lens arrangement, an LED, or the like. Detector 68 is a conventional light sensor, such as a photovoltaic sensor, which outputs a light intensity signal value.

The signal values from detector 68 are supplied to a digital processor 72 which carries out the signal-value operations described in Section C. The processor, which is also referred to herein as calculation means, is preferably designed for automated signal measuring operations, through control of actuator 64. These operations are shown in flow diagram form in Figure 10. Initially, the actuator is controlled by the processor to place the four reaction zones successively at each reaction zone, where the signal value is read and stored by processor memory (not shown).

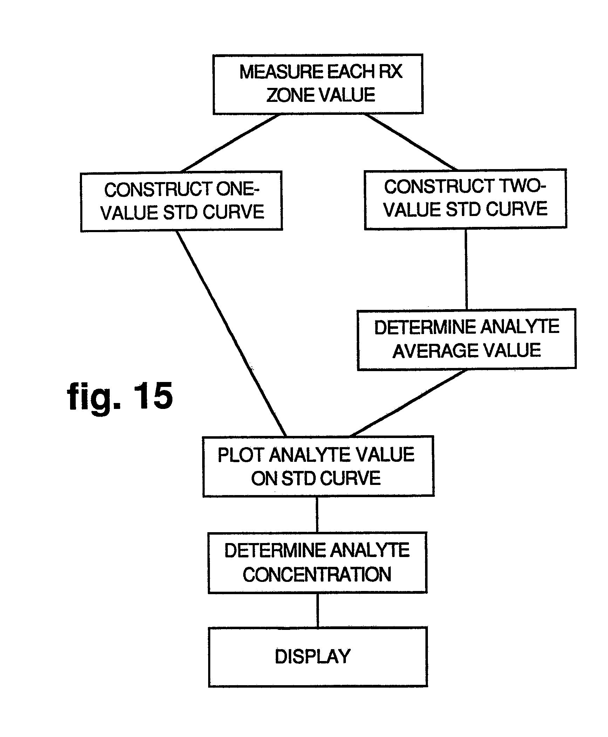

The algorithm steps shown at the left in Figure 15 are employed in the two-zone method above, where the first reaction zone provides a measure of analyte concentration, and the second zone, a measure of reference signal value. The steps performed by the algorithm involve (a) constructing a single-point standard curve from the second-zone value, (b) plotting the first-zone value on the standard curve, (c) determining analyte concentration from the standard-curve plot, and (d) displaying the desired analyte concentration value.

The algorithm steps shown at the right in Figure 15 are employed in a four-zone method above, based on a two-point standard curve, and a duplicate analyte determination. The steps performed by the algorithm involve (a) constructing a two-point standard curve from the second-and third-zone value, (b) calculating the average first- and fourth-zone values, (c) plotting the average analyte signal value on the standard curve, (d) determining analyte concentration from the standard-curve plot, and (e) displaying the desired analyte concentration value.

Processor design capable of controlling the sample holder, digitizing and storing measured reflectivity values, and carrying out the mathematical operations used to generate the standard curve functions and analyte concentration determinations from the standard curve would be well known to one skilled in the art.

The following example illustrate the use of the method for qualitatively determining total serum cholesterol. The examples is intended to illustrate, but not limit, the scope of the invention.

## Example 1

### Determining Serum Cholesterol: Two-Zone Method

An assay device like that described in Figure 1 is prepared as follows. A 150 micron thick paper filter obtained from Schleicher and Schull is cut into 2 x 4 mm rectangles. One of these rectangles, which form the upper layer of reaction zones of the device, is infused with 10 µl of a reagent solution containing 150 U/ml cholesterol ester hydrolase, 10 U/ml cholesterol oxidase, 80 U/ml peroxidase, and 20 mM 4-aminoantipyrine (4-AAP) and 80 mM 3,5-dichloro-2-hydroxybenzoic acid (DCHBS) dye, in reduced form, in deionized water. The lower layer in each reaction zone is formed of an identical-size rectangular piece cut from an 50 micron thick glass fiber filter obtained from Gelman. This layer is infused with 10 µl 0.15 mM D-proline.

A second rectangle is infused with 10µl of a reagent solution containing 25 U/ml D-amino acid oxidase, 80 U/ml peroxidase, and 20 mM 4-aminoantipyrine (4-AAP) and 80 mM 3,5-dichloro-2-hydroxybenzoic acid (DCHBS) dye, in reduced form, in deionized water. A second, lower layer is infused with 10 µl 0.15 mM D-proline, as above.

After drying under reduced pressure, the layers are placed against the associated upper-layer rectangles, and the two reaction zones are attached to opposite sides of a 50 micron thick 6 x 20 mm strip of glass fiber filter obtained from Whatman, and separated therefrom by a 1 micron pore size polycarbonate membrane.

A whole blood sample from a human test subject is obtained conventionally, and applied to the strip in an amount sufficient to wet the three reaction zones on the strip. The strip is incubated at room temperature for 90 seconds, or until no further color development is observed.

The analyte-related signal is read by reflectance spectrophotometry, at an illuminating wavelength of 500 nm. The values for the second reaction zone are compared with signal values obtained by infusing physiological saline into the second zone, i.e., where inhibition by serum components is avoided, and the percent inhibition due to serum-component inhibition is determined.

The values from the first reaction zone are used to determine serum cholesterol concentration, from a predetermined standard curve prepared by adding a predetermined volume of known-concentration solution of cholesterol in non-ionic detergent to reaction pads having the same components as the first reaction zone. The cholesterol concentration calculated from the curve is then corrected for serum inhibition effects using the second-zone inhibition value.

## Example 2

### Determining Serum Cholesterol: Three-Zone Method

The assay device is similar to that employed in Example 1, except that the three reaction zones contain the components shown in Table II above. The serum sample is introduced, and the reaction signal values are measured as in Example 1.

The values for the first, second, third and fourth reaction zones (labeled 1, 2, and 3, respectively, at the left in Figure 8), are measured at 500 nm as above. The second and third zone signal values are plotted at corresponding reference-compound concentrations, as indicated in Figure 8, yielding a standard curve which here indicates non-competitive inhibition. The signal value from the first zone is plotted on this curve, and the corrected analyte concentration is read from the corresponding position on the concentration axis.

A fourth reaction zone contains all of the components of zone 1 except cholesterol esterase. The signal value from this zone thus reflects free serum cholesterol only, since esterified cholesterol in the fluid sample is not released for use. The signal value from this zone is plotted on the two-point standard curve, as indicated in Figure 8, to determine analyte concentration for free serum cholesterol. Esterified cholesterol concentration can be determined from the signal value of (1-4).

From the foregoing, it can be appreciated how various objects and features of the invention are met. The analyte test device is simple to use, typically requiring only placement of a single fluid sample on a filter strip. Where the test device is used as part of a signal reading and calculating apparatus, the desired analyte concentration value is obtained without additional sample-handling or calculation steps on the part of the user.

The analyte concentration value obtained is based on a standard curve which is corrected for background. That is, the assay is self-corrected for age and condition of the reagents, sample composition, and reaction conditions, such as temperature, which introduce uncertainty and variability in usual strip or solid-support-type assays. Further, only two signal values are needed to calculate the corrected, standardized assay value.

Although the invention has been described with reference to particular embodiments and uses, it will be understood that various changes and modifications can be made without departing from the invention.

## Claims

1. An assay device for determining the concentration of an analyte in an aqueous fluid sample, comprising
a first reaction zone containing analyte-specific component means effective, when an analyte-containing fluid sample is added to the zone, to utilize the analyte to produce an intermediate reaction product, and signal-generating component means effective to utilize the intermediate reaction product to produce a signal reaction product having a measurable value which is dependent on the concentration of analyte present in the fluid sample, and
a second reaction zone containing a known quantity of a reference compound which is not present in such aqueous fluid sample, compound-specific component means effective, when an analyte-containing fluid sample is added to the zone, to utilize the reference compound to produce said intermediate reaction product, and said signal-generating component means effective to utilize the intermediate reaction product to produce a signal reaction product having a measurable signal value which is dependent on the concentration of reference compound present in the second reaction zone.

2. The device of claim 1, wherein the reaction zones are porous-matrix zones designed to draw the fluid sample into the zones by surface wetting, and the device further includes a reservoir in contact with the two zones, for receiving and distributing the fluid sample to the reaction zones.

3. The device of claim 1 or 2, wherein said second reaction zone is composed of two layers, one containing the reference compound, and the other, said compound-specific component means.

4. The device of any one of the preceding claims which further includes a third reaction zone which contain said reference compound, in an amount which is different from that present in the said second zone, said compound-specific component means and said signal-generating component means.

5. The device of any one of the preceding claims, wherein said analyte-specific reaction means is effective to react with analyte to produce the intermediate reaction product $H_2O_2$, said compound specific component means is effective to react with the reference compound to produce $H_2O_2$, and said signal-generating components include a peroxidase enzyme and a substrate reagent which can be converted to said signal reaction product by the peroxidase enzyme in the presence of $H_2O_2$.

6. The device of claim 6, wherein the signal-generating components further include a trapping agent effective to compete with the substrate reagent, in the presence of the peroxidase enzyme, to produce a silent reaction product which is distinguishable from the signal reaction product, with utilization of $H_2O_2$, thus proportionately reducing the amount of signal reaction product generated by a given amount of analyte.

7. The device of claim 6, wherein the trapping agent is present in the matrix in an amount which is effective to reduce the amount of said signal reaction product, as a function of analyte concentration, to about 10%-70% of the amount of signal reaction product formed in the absence of trapping agent.

8. The device of any one of claims 5-7 wherein said substrate reagent consists of primary and secondary compounds which combine to form the signal reaction product in the presence of $H_2O_2$ and peroxidase, and said trapping compound competes with the secondary compound for reaction with the primary compound, in the presence of $H_2O_2$ and peroxidase, to produce said silent reaction product.

9. The method of any one of the preceding claims, wherein said reference compound is a D-amino acid, and said compound-specific component means includes D-amino acid oxidase.

10. The device of any one of claims 1-8 for determination of serum cholesterol, wherein said analyte-specific component means includes cholesterol ester hydrolase and cholesterol oxidase.

11. The device of any one of claims 1-8 for determination of both total and free serum cholesterol, which further includes a third reaction zone containing cholesterol oxidase, but not cholesterol esterase, and said compound-specific component means.

12. Apparatus for determining the amount of an analyte in an aqueous fluid sample, comprising
an assay device for determining the concentration of an analyte in an aqueous fluid sample, comprising (a) a first reaction zone containing analyte-specific component means effective, when an analyte-containing fluid sample is added to the zone, to utilize the analyte to produce an intermediate reaction product, and signal-generating component means effective to utilize the intermediate reaction product to produce a signal product having a measurable value which is dependent on the concentration of analyte present in the fluid sample, (b) a second reaction zone containing a known amount of a reference compound which is not present in such aqueous fluid sample, compound-specific component means effective, when an analyte-containing fluid sample is added to the zone, to utilize the reference compound to produce said intermediate reaction product, and said signal-generating component means, (c) a third reaction zone containing said reference compound in an amount which is different from the amount of reference compound in the second zone, said compound-specific component means, and said signal-generating component means; and (d) means for distributing such fluid sample to each of the three reaction zones, at substantially the same analyte concentration in each reaction zone;
a signal detector for measuring such signal value in each of the three reaction zones; and
processor means for determining from the signal values of the three reaction zones (a) the relationship between reference compound concentration and measured signal value, and (b) the concentration of analyte in the first reaction zone, using such determined relationship.

13. The apparatus of claim 12, wherein said the reaction zones are porous-matrix zones designed to draw the fluid sample into the zones by surface wetting, and said distributing means includes a reservoir in contact with the three zones, for receiving and distributing the fluid sample to the reaction zones.

14. The apparatus of claim 12, or 13, wherein said signal detector includes a light source for illuminating each zone with a light beam, and a beam detector for determining the intensity of reflectance of the beam from an illuminated zone.

15. The apparatus of claim 14, wherein said processor means is designed to determine (a) a corrected relationship between reference compound concentration and measured signal value, using the signal values from measured in the second and third reaction zones, and (b) the concentration of analyte in the fluid sample, using the signal value measured in the first reaction zone and the corrected relationship between analyte concentration and measured signal value.

16. A method of determining the amount of an analyte in an aqueous fluid sample, comprising
distributing the fluid sample to first and second reaction zones, where (a) the first reaction zone contains analyte-specific component means effective, when an analyte-containing fluid sample is added to the zone, to utilize the analyte to produce an intermediate reaction product, and signal-generating component means effective to utilize the intermediate reaction product to produce a signal product having a measurable value which is dependent on the concentration of analyte present in the fluid sample, and (b) the second reaction zone contains a known amount of a reference compound which is not present in such aqueous fluid sample, compound-specific component means effective, when an analyte-containing fluid sample is added to the zone, to utilize the reference compound to produce said intermediate reaction product, and said signal-generating component means effective to utilize the intermediate reaction product to produce a signal reaction product having a measurable signal value which is dependent on the concentration of reference compound present in the second reaction zone, (c) measuring the signal values in the first and second reaction zones, (d) determining a correction factor from the measured signal value of the second reaction zone, and (e) determining from the measured signal value in the first reaction zone and the correction factor, an analyte concentration value which is corrected for inhibition and loss of activity effects in the two reaction zones.

17. The method of claim 16, for qualitative determination of analyte concentration, wherein said signal values are color entities, said correction factor is represented by a percent reduction in expected optimal color intensity in the second zone, and determining the corrected analyte concentration includes adding the correction factor to an uncorrected analyte concentration determined from the color intensity in said first zone.

18. The method of claim 16, for qualitative determination of analyte concentration, wherein the measured signal value from the second reaction zone is used to construct a single-point standard curve, and the corrected analyte concentration is determined by plotting the signal value from the first reaction zone on the curve.

19. The method of claim 16, for qualitative determination of analyte concentration, wherein said fluid sample is also distributed to a third reaction zone which is like the second zone, but contains a different known amount of reference compound, wherein the signal values measured from the second and third zones are used to construct a two-point standard curve, and the corrected analyte concentration is determined by plotting the signal value from the first reaction zone on the curve.

20. The method of claim 19, for use in determining serum cholesterol, wherein said analyte-specific component means includes cholesterol ester hydrolase and cholesterol oxidase, for producing the intermediate reaction product $H_2O_2$ in the presence of cholesterol analyte, said compoundspecific component means is effective to react with the reference compound to produce $H_2O_2$ and said signal-generating component means includes a peroxidase, and a dye which is converted by the peroxidase, in the presence of $H_2O_2$, to a distinctively colored, signal reaction product.

fig. 1

fig. 2

fig. 3

fig. 4

fig. 5

EP 0 357 400 A2

fig. 6

Competitive inhibition

Non-competitive

CONC.

fig. 7

STD          STD          CONC.

fig. 8

STD    CH-FREE    STD $\Sigma$CH    CONC.

EP 0 357 400 A2

**fig. 9**

analyte

↓ analyte reagent

$H_2O_2$

substrate
reagent — peroxidase — trapping agent

signal rx product          silent rx product

cholesterol-ester

↓ cholesterol esterase

cholesterol

**fig. 10**    ↓ cholesterol oxidase

$H_2O_2$

secondary
dye — peroxidase — trapping agent
primary dye

colored product          colorless product

fig. 11

4-AMINO ANTIPYRINE
(4-AAP)

N-ETHYL-N-SULFOHYDOXY
PROPYL-M-TOLUIDINE
(TOOS)

$H_2O_2$
PEROXIDASE

fig. 12

IMINE QUINONE DYE

**4-AMINO ANTIPYRINE (4-AAP)**

**COLORLESS PRODUCT**

fig. 13

fig. 14

fig. 15